# EUROPEAN PATENT SPECIFICATION

(11) **EP 3 542 165 B1**
(45) Date of publication and mention of the grant of the patent: **05.01.2022**
(21) Application number: 17801707.5
(22) Date of filing: 21.11.2017
(51) Int. Cl.: G01N 33/68, G01N 21/552

(54) **COMBINED ASSAY FOR THE DIFFERENTIAL DIAGNOSIS OF THE ALZHEIMER'S DISEASE**
KOMBINATIONS-ASSAY FÜR DIE DIFFERENZIALDIAGNOSE DER ALZHEIMER-KRANKHEIT
DOSAGE COMBINÉ POUR LE DIAGNOSTIC DIFFÉRENTIEL DE LA MALADIE D'ALZHEIMER

(30) Priority: 21.11.2016 EP 16199805
(43) Date of publication of application: 25.09.2019
(73) Proprietor: betaSENSE GmbH, 48147 Münster (DE)
(72) Inventor: GERWERT, Klaus, 48147 Münster (DE); NABERS, Andreas, 85570 Markt Schwaben (DE); SCHARTNER, Jonas, 38350 Helmstedt (DE)
(74) Representative: Huenges, Martin
(86) International application number: PCT/EP2017/079949
(87) International publication number: WO 2018/091743

(56) References cited:
- EP-A1- 2 700 933
- WO-A1-2015/121339
- ANDREAS NABERS ET AL: "Amyloid-[beta]-Secondary Structure Distribution in Cerebrospinal Fluid and Blood Measured by an Immuno-Infrared-Sensor: A Biomarker Candidate for Alzheimer's Disease", ANALYTICAL CHEMISTRY, vol. 88, no. 5, 1 March 2016 (2016-03-01), pages 2755-2762, XP055352690, US ISSN: 0003-2700, DOI: 10.1021/acs.analchem.5b04286 cited in the application -& Andreas Nabers ET AL: "Amyloid-beta-Secondary Structure Distribution in Cerebrospinal Fluid and Blood Measured by an Immuno-IR-Sensor: A Biomarker Candidate for Alzheimer's Disease - SUPPLEMENT", Analytical Chemistry, 1 March 2016 (2016-03-01), pages S-1, XP055352696, Retrieved from the Internet: URL:http://pubs.acs.org/doi/suppl/10.1021/ acs.analchem.5b04286/suppl_file/ac5b04286_ si_001.pdf [retrieved on 2017-03-08]
- ANDREAS NABERS ET AL: "An infrared sensor analysing label-free the secondary structure of the Abeta peptide in presence of complex fluids", JOURNAL OF BIOPHOTONICS, vol. 9, no. 3, 24 March 2015 (2015-03-24), pages 224-234, XP055352691, DE ISSN: 1864-063X, DOI: 10.1002/jbio.201400145

## Description

The invention provides a combined immuno-infrared assay for the differential diagnosis and sub-classification of Alzheimer's disease into different disease stages. The method can be applied for assured disease diagnostics and patient stratification. The assay considers the label-free detection of the change within the Amyloid-beta peptide and Tau protein secondary structure distribution in bodily fluids. This secondary structure change from native to β-sheet enriched isoforms occurs time-delayed for Aβ and Tau, but appears years before clinical disease manifestation. Now, the combined method utilizes this shift for diagnostics based on liquid biopsies.

### Background of the invention

Alzheimer's disease (AD) is one of the most frequent neurodegenerative diseases which affects over 35 million people worldwide (Prince et al., London, UK doi:10.1111/j.0963-7214.2004.00293.x (2015)). The differential diagnosis and sub-classification of AD, especially into early or prodromal stages of disease, is still challenging in clinical routine. The need for reliable biomarkers for the early detection of AD is currently in demand. But assured and early differential diagnostics are fundamental for future therapeutic interventions (Chiba, Neurodegenerative Diseases, edited by Uday Kishore, 181-225. InTech doi:10.5772/55293 (2013); Thorsett and Latimer, Current Opinion Chem. Biol. 4(4):377-82 (2000)). Therefore, scientific research institutes are focusing on simple diagnostic tests preferably based on liquid biopsies (Doecke, Arch. Biol. 69(10):1318 doi:10.1001/archneurol.2012.1282 (2012); Andreasen et al., J. Neurology, Neurosurgery and Psychiatry 64(3):298-305 (1998); Fiandaca et al., Frontiers in Neurology 6(Nov):1-13 doi:10.3389/fneur.2015.00237 (2015); Mapstone et al., Nature Medicine 20(4):415-18. doi:10.1038/nm.3466 (2014)).

In Alzheimer's disease a secondary structure change of the mostly intrinsic disordered Amyloid-beta (Aβ) peptide and Tau protein into β-sheet enriched isoforms is discussed as an initiating event during the disease progression (Sarroukh et al., Cell. Mol. Life Sci. 68(8):1429-38 doi:10.1007/s00018-010-0529-x (2011); Cerf et al., Biochem. J. 421(3):415-23 doi:10.1042/BJ20090379 (2009); Fändrich, et al., Prion 3(2):89-93 (2009); Sachse et al., PNAS 105(21):7462-66 doi:10.1073/pnas.0712290105 (2008); Glabe, J. Biol. Chem. 283(44):29639-43 doi:10.1074/jbc.R800016200 (2008); Cavallucci et al., Mol. Neurobiol. doi:10.1007/s12035-012-8251-3 (2012); Haass and Selkoe, Nature Rev. Mol. Cell Biol. 8(2):101-12 doi:10.1038/nrm2101 (2007); Kolarova et al., Int. J. Alzheimer's Disease doi:10.1155/2012/731526 (2012); Yang et al., Devel. Brain Res. 156(2):127-38 doi:10.1016/j.devbrainres.2005.02.004 (2005)). Thereby, Tau aggregation and deposition into neurofibrillary tangles (NFT) is suggested to accompany Aβ aggregation (Lo et al., Arch. Neurol. 68(10):1257-66 doi:10.1001/archneurol.2011.123 (2011); Bennett et al., Arch. Neurol. 61(3):378-84 doi:10.1001/archneur.61.3.378 (2004); Coomaraswamy et al., PNAS 107(17):7969-74 doi:10.1073/pnas.1001056107 (2010); Braak and Braak, Acta Neuropathologica 82(4):239-59 doi:10.1007/BF00308809 (1991); Thal et al., J. Neuropath. Exp. Neurol. 59(8):733-48. (2000); Thal et al., Science of Aging Knowledge Environment 2006(6):re1 doi:10.1126/sageke.2006.6.re1 (2006)).

In clinical routine neuropsychological tests and neurochemical quantitative results of diverse biomarker levels in cerebrospinal fluid (CSF) are used for state of the art differential diagnostics. But biomarker concentrations itself, like Aβ40, Aβ42, the total Tau or hyperphosphorylated Tau level, might not correlate with AD progression (Wiltfang et al., J Neurochem., 101(4):1053-59 doi:10.1111/j.1471-4159.2006.04404.x (2007), Gabelle et al., J Alzheimers Dis., 26(3):553-63 doi:10.3233/JAD-2011-110515 (2011), Blennow et al., J Nutr Health Aging, 13(3):205-8 doi:10.1007/s12603-009-0059-0 (2009)). Moreover, based on these biomarker quantification differential diagnostics remain challenging. However, Positron emission tomography (PET) and Magnetic resonance tomography (MRT) detect aggregates (accumulated from β-sheet enriched proteins) such as plaques in the human brain. Nevertheless, PET and MRT are very expensive and time-consuming techniques, which are not applicable for the detection of prodromal AD stages and thus provide only the determination of moderate/late stages of the disease. A further disadvantage is in the case of PET the usage of contrast agents, which also stress the patients. Besides the already mentioned techniques, fluorescence based immuno assays are an emerging field, especially Enzyme Linked Immunosorbent Assay (ELISA) and surface-based fluorescence intensity distribution analysis (sFIDA). But these techniques need fluorescence labeled detection antibodies, which can influence the secondary structure of the analyzed biomarker. Moreover, ELISA and sFIDA did not reveal any direct information on the protein secondary structure or the secondary structure distribution. Furthermore, the secondary structure of the Tau protein was never used for diagnostic or differential purposes to date because of the missing conformational sensitivity of the mentioned techniques.

In order to determine such secondary structure change Fourier-transform infrared-(FTIR-) difference-spectroscopy is a powerful tool (Kötting and Gerwert, Chemphyschem 6(5):881-888 doi:10.1002/cphc.200400504 (2005)). The frequency of the amide I band caused by the C=O vibration of peptide bond is indicative for the secondary structure of the protein backbone. Especially, the increase of β-sheet enriched biomarker isoforms in bodily fluids is reliably detected by a frequency downshift to 1630 cm⁻¹ monitored by the surface probing attenuated total reflection (ATR) technique. In order to analyze the secondary structure distribution of a specific protein in bodily fluids, the protein of interest has to be selectively bound within the surface layer, which is achieved with an antibody-functionalized internal reflection element (IRE) (Schartner et al., JACS 135(10):4079-87 doi:10.1021/ja400253p (2013)). This method was applied for the extraction and determination of the secondary structure distribution of the soluble Aβ fraction from CSF and blood plasma for moderate AD and disease control differentiation (Nabers et al., J. Biophotonics 9(3):224-34 doi:10.1002/jbio.201400145 (2016); Nabers et al., Anal. Chem. Doi: 10.1021/acs.analchem.5b04286 (2016)).

In contrast, techniques like surface plasmon resonance (SPR), surface acoustic waves or quartz crystal microbalance are used to analyse protein-ligand or protein drug interactions. Since, these techniques only provide kinetical information, but no spectral resolution, they are not able to reveal a direct secondary structural change within a protein. Further techniques like surface enhanced Infrared absorption (SEIRA) spectroscopy would in theory provide spectral resolution, but the reproducibility of the measurements is very challenging due to the preparation of the rough gold surfaces and thus does not provide a robust platform for protein secondary structural transition analysis.

WO 2015/121339 provides a biosensor for conformation and secondary structure analysis, notably for the direct non-invasive qualitative secondary structure analysis of a single selected protein within a complex mixture, as e.g. a body fluid, by vibrational spectroscopic methods. For the analysis it is not required that the selected substance is isolated, concentrated, or pretreated by a special preparative procedure. The biosensor is suitable for the determination of progression of a disease, in which a conformational transitions of a candidate biomarker protein is associated with disease progression, wherein a shift of the amide I band maximum of the biomarker protein is a classifier indicative for the progression of the disease. Considering protein misfolding diseases as e.g. Alzheimer's disease, Parkinson's disease, Creutzfeldt-Jakob disease, or Huntington's disease, this information is crucially connected to the disease progression.

### Short Description of the Invention

The subject matter of the invention is as defined in the appended claims.

Alzheimer's disease (AD) is a multifactorial proteopathy including the misfolding of two prominent biomarker candidates. Both, the Amyloid-beta peptide (Aβ) and Tau protein, show enhanced β-sheet isoforms during the disease progression. Previously, an increased content of β-sheet Aβ isoforms in the total Aβ fraction in cerebrospinal fluid (CSF) and blood plasma could be applied for AD detection by an immuno-infrared sensor. Here, 300 samples from disease control (DC) and Dementia Alzheimer type (DAT) patients were analyzed in regard to the secondary structure distribution of soluble Aβ (CSF, blood plasma) and the Tau protein (CSF), respectively. The Tau protein secondary structure distribution proved to be a general marker of dementia, not specifically for DAT, but a combined data analysis of Aβ and Tau yielded a diagnostic assay for DC/DAT differentiation with an accuracy of 93 %. Moreover, the combined data evaluation showed the potential to subdivide DAT patients in early and late stages of DAT and may provide a differential diagnosis of DC subjects.

### The invention thus provides

(1) a method for the differential diagnosis and sub-classification of Alzheimer's disease into different disease stages by direct analysis of the secondary structure distribution of a soluble Amyloid-beta (Aβ) peptide fraction and a soluble Tau protein fraction in bodily fluids, comprising the steps
   (a) conducting, in a first IR cell comprising a first infrared sensor element having an internal reflection element with a core of an infrared transparent material and at least one receptor for the Aβ peptide directly grafted to at least one surface of said core, said at least one receptor for the Aβ peptide being antibodies capable of specific and conformationally independent binding to the Aβ peptide, at least one flux of a body fluid with soluble Aβ peptide, submitting an IR beam through said first IR cell, and obtaining an infrared spectrum therefrom;
   (b) conducting, in a second IR cell comprising a second infrared sensor element having an internal reflection element with a core of an infrared transparent material and at least one receptor for the Tau protein directly grafted to at least one surface of said core, said at least one receptor for the Tau protein being antibodies capable of specific and conformationally independent binding to the Tau protein, at least one flux of a body fluid with soluble Tau protein, submitting an IR beam through said second IR cell, and obtaining an infrared spectrum therefrom; and
   (c) analyzing the obtained infrared spectra to determine the secondary structure distribution of the soluble Aβ peptide and of the soluble Tau protein in the bodily fluids for the differential diagnosis, wherein a downshift of the amide I band of the Aβ peptide and of the Tau protein is indicative for the disease stage.

The invention further provides a kit for the differential diagnosis and sub classification of Alzheimer's disease into different disease stages comprising a first and second infrared sensor element according to the present invention.

The invention also provides a device for the differential diagnosis and sub classification of Alzheimer's disease into different disease stages, said device comprising a first and second infrared sensor element according to the present invention.

The invention further provides a use of the first and second infrared sensor element according to the present invention, the kit according to the present invention or the device according to the present invention for direct analysis of the secondary structure distribution of a soluble Amyloid-beta (Aβ) peptide fraction and a soluble Tau protein fraction in bodily fluids.

### The invention thus provides

(2) a preferred embodiment of aspect (1) above, wherein said first and second infrared sensor elements comprise a germanium internal reflection element being of trapezoid or parallelogram shape,

(3) a kit for the differential diagnosis and sub-classification of Alzheimer's disease into different disease stages comprising a first and second infrared sensor element as defined in (1) or (2) above,

(4) a device for the differential diagnosis and sub-classification of Alzheimer's disease into different disease stages, said device comprising a first and second infrared sensor element as defined in (1) or (2) above, and

(5) the use of the first and second infrared sensor element as defined in (1) or (2) above, the kit as defined in (3) above or the device as defined in (4) above for direct analysis of the secondary structure distribution of a soluble Amyloid-beta (Aβ) peptide fraction and a soluble Tau protein fraction in bodily fluids.

The present invention is based on the separate detection of Aβ and Tau with two sensor elements. In this context, the analysis and sub-classification bases on the determination of the secondary structure distribution of Aβ and Tau both extracted separately from bodyly fluids. Up to now, the secondary structure distribution of the Tau protein in CSF has never been considered for diagnostic purposes. Moreover, including the secondary structure change of Tau out of CSF for Alzheimer's disease detection provides more than an additive effect on the diagnostic accuracy. Analyzing the secondary structure distribution of Aβ (e.g. in CSF and/or blood plasma), and Tau (e.g. in CSF) enables the sub-classification of Alzheimer's disease in mild to severe disease stages, and the differentiation between AD and other dementia types.

### Short Description of the Figures

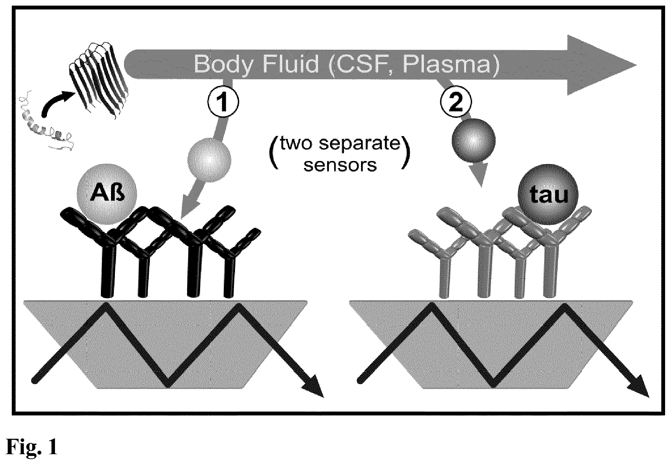

Figure 1: Scheme of the combined immuno-infrared assay and principle of the analysis. (A) The total fraction of Aβ (1) and Tau (2) present in CSF and/or plasma were separately extracted using an antibody functionalized immuno-infrared sensor. The detected Aβ and Tau secondary structure distribution is indicated by the infrared amide I maximum position.

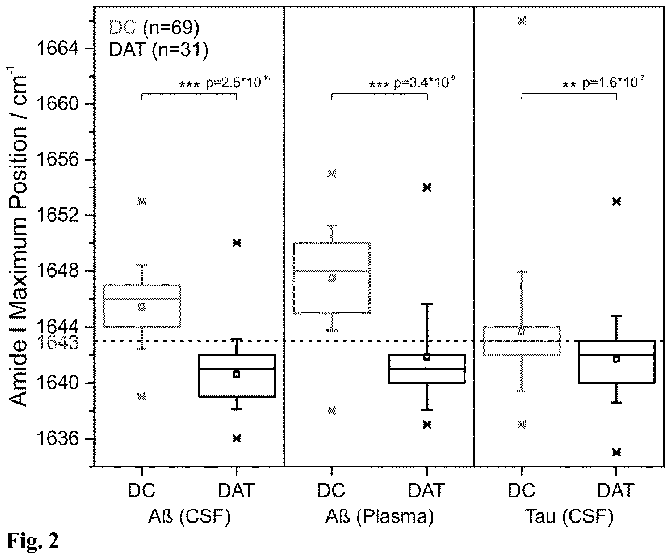

Figure 2: Distribution of the amide I maximum position as displayed in box-plots for DC and DAT discrimination based on the analysis of Aβ in CSF and plasma, and Tau in CSF. Both diagnostic groups showed a high significant difference in the amide I maximum of Aβ out of CSF (p=2.5^{∗}10⁻¹¹, Kruskal-Wallis ANOVA, confidence level a=0.05) and EDTA-plasma (p=3.4^{∗}10⁻⁹), and a moderate significance for Tau out of CSF (p=1.6^{∗}10⁻³). In Box-plots 25/50/75% quantiles are displayed as horizontal lines, the average band position as square, ± standard deviation as whiskers, and observed minimum/maximum values as cross.

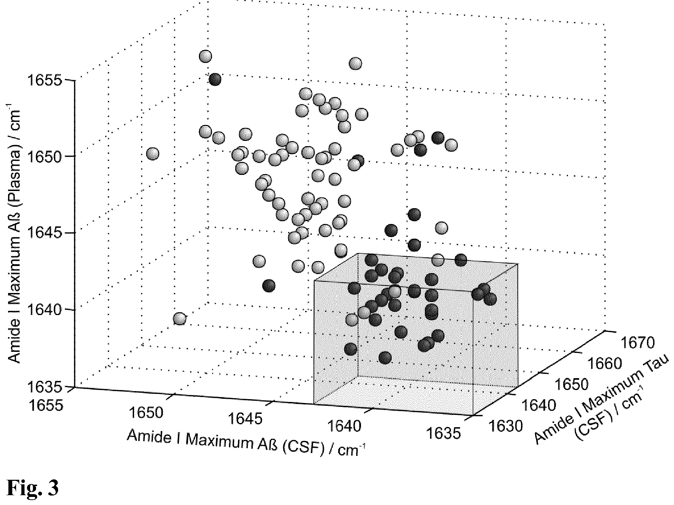

Figure 3: 3D-scatter plot of the amide I maximum position as determined for Tau in CSF, Aβ in CSF and Aβ in EDTA-plasma for 61 DC (grey) and 39 DAT (black) samples. Data points within the transparent black box indicate subjects which are identified as DAT in all three assays.

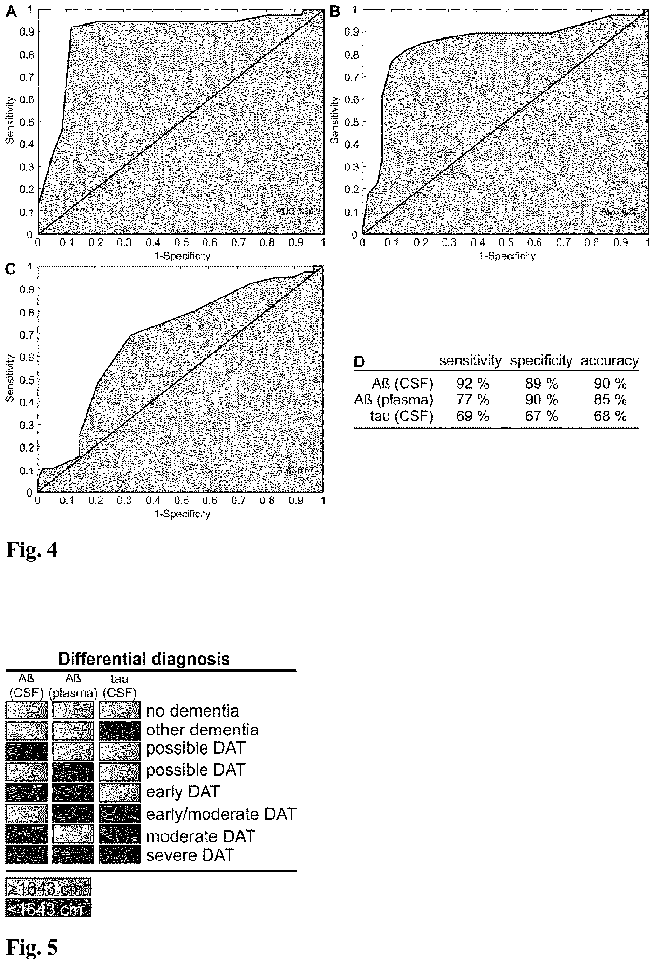

Figure 4: ROC-curve analysis for DC (n=61) and DAT (n=39) differentiation based on the determination of the Aβ secondary structure distribution in CSF (A), Aβ in EDTA-plasma (B), and Tau protein in CSF (C). In this order an AUC of 0.90, 0.85, and 0.67 was achieved. Thus, a diagnostic accuracy of 92 % (Aβ, CSF), 85 % (Aβ, plasma), and 68 % (Tau, CSF) was calculated (D). Based on these data, the Tau secondary structure distribution alone seems more to be a general biomarker for dementia than for the specific DAT detection.
Figure 5: Diagram of the procedure for the differential diagnosis and disease stage classification of DAT and other types of dementia by the combined immuno-infrared assay. The assay is based on the determination of the Aβ peptide and Tau protein secondary structure distribution in bodily fluids. This distribution is represented by the maximum position of the infrared conformation sensitive amide I band of the extracted biomarker fraction. A maximum below the discriminative marker band of 1643 cm⁻¹ is defined as diseased. This procedure is applied to the extracted Aβ fraction out of CSF and plasma and to the Tau protein fraction from CSF. No dementia will be assigned when all three biomarker values are above or equal to 1643 cm⁻¹. In contrast, biomarker values below 1643 cm⁻¹ indicate severe DAT. Other types of dementia will be identified when only the Tau amide I maximum is below the marker band.

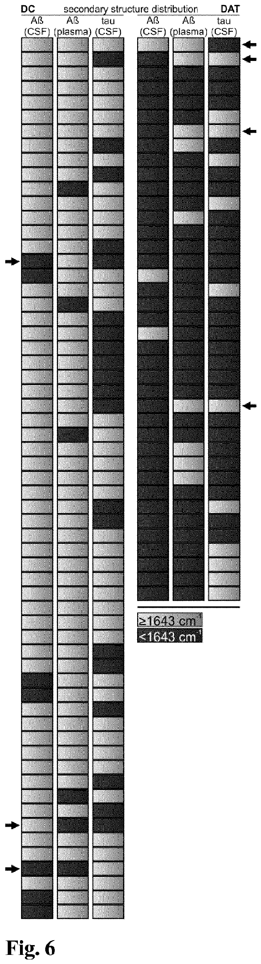

Figure 6: The determination of the amide I maximum representing the secondary structure distribution of Aβ (CSF/plasma) and Tau (CSF) was used for the differentiation of 61 DC and 39 DAT patients. Thereby, a majority vote (black = maximum < 1643 cm⁻¹ and grey = maximum ≥ 1643 cm⁻¹) depicted DC and DAT. Thus, only 3 false positives were observed for the DC group and 4 for the DAT group. This results in a specificity of 95 %, sensitivity of 90 %, and thus an overall diagnostic accuracy of 93 % for the combined data analysis as compared to the clinical assessment by geronto psychiatrists and neurologists.

### Detailed Description of the Invention

The immuno-infrared sensors and their production is described in applicant's previous patent application WO 2015/121339 and which is now applied for the detection of the secondary structure distribution of both Aβ and Tau in bodily fluids. The production of the IR-sensors includes the direct and intimate immobilization of receptors for the Aβ or Tau, respectively, i.e. antibodies, on the surface of the infrared transparent material via silane or thiol chemistry with an optimized, simplified protocol. To analyze the liquid (e.g. serum, blood plasma or CSF), it is fed to the sensor in a flow system. The macromolecular substance is immobilized by the antibody on the functionalized sensor surface. The optical sensor elements are particularly suitable for infrared analysis and optionally further for the parallel or alternative analysis by another optical method including detection of fluorescence at different wavelengths.

According to the invention, the infrared transparent material of the first and second IR cells is independently selected from silicon, germanium, zinc selenide, gallium selenide, and diamond, and preferably is germanium.

In a preferred embodiment of the invention, the optical sensor elements has an internal reflection element comprising a germanium crystal having a trapezoid or parallelogram shape, fiber or rod shaped geometry. It is preferred that the germanium crystal is a germanium monocrystal, while a trapezoid cut germanium monocrystal is particularly preferred.

It is further preferred that the germanium crystal allows for or provides for one, more than one, or more than three reflections of the infrared light through the reflection element, particularly preferred are more than five reflections or even more than twenty reflections (preferred are 25 reflections with 13 actively sensed reflections). For allowing the contact with the candidate biomarker protein in such multiple reflections, the receptor for the biomarker protein is grafted to the appropriate number of surfaces of said internal germanium reflection element.

The silane and thiol linkers that can be utilized for coupling the receptor and hence, the macromolecule to the internal germanium reflection element include homogenous silane and thiol linkers, mixtures of silane linkers and mixtures of thiol linkers. For allowing a tight and intimate linkage of the receptor/macromolecule short chained linkers, preferably linkers having an effective linker chain length (including carbon atoms and heteroatoms) of not more than 20 atoms or not more than 15 atoms, can be utilized.

Such short chained linkers include silane linkers that have one of the following formulas:

X₃Si-(CH₂)ₙ-Y-(CH₂)_{n'}-Z,

X₂R¹Si-(CH₂)ₙ-Y-(CH₂)_{n'}-Z or

X(R¹)₂Si-(CH₂)ₙ-Y-(CH₂)_{n'}-Z,

and the thiol linkers have the following formula:

WS-(CH₂)ₙ-Y-(CH₂)_{n'}-Z, wherein W is R¹S- or H, X at each occurrence is independently selected from halogen and C₁₋₆ alkoxy, n is an integers of 1 to 10, n' is an integer of 1 to 5, R¹ at each occurrence is independently selected from C₁₋₆ alkyl, Y is selected from a chemical bond, -O-, -CO-, -SO2-, -NR²-, -S-, -SS-, -NR²CO-, -CONR²-, -NR²SO2- and -SO₂NR²- (wherein R² is H or C₁₋₆ alkyl), and Z is an amine-reactive group including -CO₂H, -SO₃H and ester derivatives thereof.

The halogen includes a fluorine, chlorine, bromine and iodine atom. C₁₋₆ alkyl and C₁₋₆ alkoxy includes straight, branched or cyclic alkyl or alkoxy groups having 1 to 6 carbon atoms that may be saturated or unsaturated. In case of cyclic alkyl and alkoxy groups, this refers to those having 3 to 6 carbon atoms. Suitable C₁₋₆ alkyl and C₁₋₆ alkoxy groups include, among others, methyl and methoxy, ethyl and ethoxy, n-propyl and n-propoxy, iso-propyl and iso-propoxy, cyclopropyl and cyclopropoxy, n-butyl and n-butoxy, tert-butyl and tert-butoxy, cyclobutyl and cyclobutoxy, n-pentyl and n-pentoxy, cyclopentyl and cycloppentoxy, n-hexyl and n-hexoxy, cyclohexyl and cyclohexoxy, and so on. The amine-reactive group Z includes all types of functional groups that are reactive with a free amino group. Among those, - CO₂H, -SO₃H and ester derivatives thereof (including active esters) are particularly preferred.

The -(CH₂)ₙ- and -(CH₂)_{n'}- structural elements in the above formulas may also contain one or more double and/or triple bonds and may be substituted with one or more halogen atoms such as fluorine or with deuterium.

The optical sensor elements are obtainable by silanization and in the linkers of formulas (i) to (iii) X is independently selected from C₁₋₆ alkoxy groups, preferably from methoxy and ethoxy groups, Y is -NHCO-, Z is -CO₂H or an ester derivative thereof, and n is an integer of 1 to 5 and n' is an integer of 1 to 3, preferably n is 3 and n' is 2.

Alternatively, the optical sensor elements are obtainable by thiolation and in the linkers of formula (iv) W is H, Y is a chemical bond, Z is -CO₂H or an ester derivative thereof, and n is an integer of 1 to 8 and n' is an integer of 1 to 5, preferably n is 8 and n' is 4. Particularly preferred is a 12-mercaptododecanoic acid NHS ester.

According to the invention, the receptors for the Aβ peptide and Tau protein are specific antibodies. In case of the Aβ peptide, the antibody is an antibody specifically binding to the central epitope of the Aβ peptide, such as antibody A8978 (Sigma Aldrich) and in case of the Tau protein, the antibody is an antibody specifically binding to an epitope present in all Tau variants (including phosphorylated and truncated variants, variants with 3 to 4 repeat regions, or isoforms), such as antibody Tau-5 (AHB0042, Thermo Fisher Scientific).

The blocking substance not cross-reacting with the candidate biomarker protein includes casein, ethanolamine, L-lysine, polyethylene glycols, albumins, and derivatives thereof, and preferably is casein.

In a method for preparing the sensor elements, the oxidization is performed by treatment with H₂O_{2/}oxalic acid. Further, in the method the silanization with the short silane linkers is preferably performed with a silane derivative having the following formulas:

X₃Si-(CH₂)ₙ-(CH₂)_{n'}-Y,

X₂(R¹)Si-(CH₂)ₙ-(CH₂)_{n'}-Y or

X(R¹)(R²)Si-(CH₂)ₙ-(CH₂)_{n'}-Y,

wherein the variables are as defined above. It is particularly preferred that an ester derivative of the CO₂H or SO₃H moiety in the definition of Y be used, which can be a simple C₁₋₆ alkyl ester, but can also be an activated ester such as an N-hydroxysuccinimid ester or any other activated ester derivate.

It is further preferred that the blocking substance is casein.

In a method for preparing the sensor elements, the surface activation is performed by treatment with HF (49%). Further, in the method the thiolation with the short thiol linkers is preferably performed with thiol linkers having the following formula:

WS-(CH₂)ₙ-Y-(CH₂)_{n'}-Z,

wherein the variables are as defined above. It is particularly preferred that an ester derivative of the CO₂H or or SO₃H moiety in the definition of Y be used, which can be a simple C₁₋₆ alkyl ester, but can also be an activated ester such as an N-hydroxysuccinimid ester or any other activated ester derivate. It is further preferred that the blocking substance is casein.

In a method for preparing the sensor elements, the optical sensor elements are built up under room temperature. Every single step can be assessed on the basis of the IR-spectra. This validation step is essential for the specific detection and accurate secondary structure determination of the analyte.

The device of aspect (4) of the invention has the sensor elements incorporated in a suitable IR cell (chamber). It may further include a light (IR) emitting element, a light (IR) detecting element and a data processing unit. For parallel detection by an additional optical method the device may further include light source and detector element for such additional optical method such as light source and detector elements for UV/Vis-fluorescence, at different wavelengths.

The method of aspect (1) of the invention comprises the steps as defined above.

In the method of the invention, the bodily fluids applied in steps (a) and (b) may be any complex body fluid comprising the biomarker, including serum, blood plasma and CSF. Further suitable bodily fluids are lacrimal fluid and nipple aspirate fluid.

In a preferred embodiment the method further comprises prior to step (a) and (b): installation of said optical sensor element in the IR cell. Additionally/alternatively the method may further comprise the step (a') and (b'): regenerating of the surface of the optical element by application of a solution of free ligand for the receptor.

The spectrum obtained in steps (a) and (b) have a sufficient signal to noise ratio to resolve the amide I band. This allows the analysis of the shift of the amide I band maximum of the biomarker protein in step (c) to determine the secondary structure of the candidate biomarker proteins and perform the differential diagnosis.

In a further embodiment the step (c) of the method further comprises comparing the obtained infrared spectrum with a spectrum of the soluble Aβ peptide and/or of the soluble Tau with known secondary structure and/or with known concentration.

In another embodiment, the method may comprise, alternative or parallel to the infrared analysis, detection by another optical method, including UV/Vis-fluorescence, at different wavelengths. Notably, a method is preferred that combines immuno-ATR-FTIR vibrational spectroscopy with parallel fluorescence spectroscopy.

The method of aspects (1) allows/is suitable for determining the soluble Aβ peptide and the soluble Tau in bodily fluids, notably for directly determining them in bodily fluids of mammalian (human, animal) origin, including cerebrospinal fluid, blood or serum, without pretreatment (i.e., without a separate preceding enrichment or purification step). The method is suitable for determination of the candidate biomarker protein in a separate (*in-vitro*) or an online (direct determination of the body fluid on the patient) fashion. In both cases, the method may further comprise the differential diagnosis and the assessment of the Alzheimer's disease stages.

The method of aspect (1) are particularly suitable for the determination of progression of Alzheimer's disease with Amyloid-beta and Tau as candidate biomarker proteins, wherein a shift of the amide I band maximum of the Aβ peptide from 1647 cm⁻¹ to 1640 cm⁻¹, preferably with a threshold value of 1643 cm⁻¹ +/- 5 cm⁻¹, (or 1643 cm⁻¹ +/- 3 cm⁻¹, or 1643 cm⁻¹ +/- 1 cm⁻¹ , or about 1643 cm⁻¹), and a shift of the amide I band maximum of the Tau protein from 1647 cm⁻¹ to 1640 cm⁻¹, preferably with a threshold value of 1643 cm⁻¹ +/- 5 cm⁻¹, (or 1643 cm⁻¹ +/- 3 cm⁻¹, or 1643 cm⁻¹ +/- 1 cm⁻¹ , or about 1643 cm⁻¹) are indicative for Alzheimer's disease. The method is also particularly suitable for the determination of progression of Alzheimer's disease with Amyloid-beta and Tau as candidate biomarker proteins. Here the differential diagnosis provides for an assured clinical profile of the dementia type, preferably the method comprises the detection of the secondary structure distribution of Aβ from CSF (A), Aβ from blood plasma (B), and Tau from CSF (C). In particular, the method of the invention enables the differential diagnosis of Dementia Alzheimer type (DAT) and (Disease Control), DAT patients being sub-classified into early, moderate, and severe DAT, and DC patients being separated into health controls, other diseases, and dementia due to another origin than Alzheimer's disease. Notably, for both biomarkers, (A)/(B) for Aβ and (C) for Tau, a discriminative threshold (1643 cm⁻¹ ± 5 cm⁻¹) separates Alzheimer's disease and DC patients; and/or the combination of (A), (B), and (C) provides a biomarker panel applicable for an assured DAT diagnosis.

A simple threshold classifier is established for both biomarkers similar to that described in Nabers et al., Anal. Chem. Doi: 10.1021/acs.analchem.5b04286 (2016) and WO 2015/121339. Thereby, using a discriminative spectral marker band for disease control (DC) and the Dementia Alzheimer type (DAT) differentiation, both diagnostics groups could be separated with a diagnostic accuracy of 90 % based on CSF Aβ analysis. The predicitve accuracy observed from blood plasma Aβ anaylsis solely was lower (84 %). Moreover, a separation of both groups only based on the Tau protein secondary structure distribution remained insufficient with an accuracy of 68 %. But combining the determined amide I frequencies of Aβ from CSF and blood plasma with those of Tau to a marker panel, a simple majority vote classifier demonstrated significant higher predicitve values. Now, an accuracy of 93 % and a specificity of 95 % could be achieved. A high specificity is cruical especially for incurable diseases such as AD, because a false positive diagnosis may have serious psychological consequences for the party concerned. But the combined data analysis demonstrated a second big advantage. By combining the data of Aβ and Tau, more information about the disease stage and indications for other types of dementia can be provided. The principle for differential diagnostics is simple. In a first step, the amide I maximum of the extracted soluble fraction of Aβ from CSF was determined as described in Nabers et al., in Anal. Chem Doi: 10.1021/acs.analchem.5b04286 (2016) and WO 2015/121339. Thereby, a maximum above or equal to 1643 cm⁻¹ was indicative for DCs, a maximium below this frequency for DAT. In a second step, the same procedure was applied to blood plasma samples. Again, the amide I maxima of Aβ were determined for each sample. Now, if both values were consistently above or below the marker frequency, a differentiation between DC and DAT could be made with a high accuracy. But for inconsistent Aβ results (CSF and plasma) the Tau protein secondary structure distribution could be used as decision support. On the other hand, the Tau protein secondary structure distribution also demonstrated the potential to sub-classify the DC and DAT group into dementia due to another origin than Alzheimer or into an early, moderate, or severe stage of the disease. Therewith, Parkinson disease or vascular dementia patients could be identified within the DC group (Aβ CSF ≥1643 cm⁻¹; Aβ plasma ≥1643 cm⁻¹; Tau CSF <1643 cm⁻¹). On the other hand, the DAT group could be differentiated into early (f.e. Aβ CSF <1643 cm⁻¹; Aβ plasma <1643 cm⁻¹; Tau CSF ≥1643 cm⁻¹), moderate (Aβ CSF <1643 cm⁻¹; Aβ plasma ≥1643 cm⁻¹; Tau CSF <1643 cm⁻¹) or (Aβ CSF ≥1643 cm⁻¹; Aβ plasma <1643 cm⁻¹; Tau CSF <1643 cm⁻¹), and severe (Aβ CSF <1643 cm⁻¹; Aβ plasma <1643 cm⁻¹; Tau CSF <1643 cm⁻¹) stages of disease.

The invention is further described by the following examples which are, however, not to be construed as limiting the invention.

### Examples

Materials and Methods: The same experimental set-ups were used as in WO 2015/121339.

Sampling and pretreatment: CSF was drawn by lumbal puncture and aliquoted at the university hospital Essen, snap-frozen in liquid nitrogen, shipped and stored at -80 °C. Samples were not pretreated before the measurement, only thawed at 37 °C for 30 seconds and kept on ice until used.

Patient collective: Details of sample acquisition and diagnosis of Disease Control (DC) and Dementia Alzheimer type (DAT) patients have been described in detail previously (Nabers et al., Anal. Chem. Doi: 10.1021/acs.analchem.5b04286 (2016)). In the former study, 141 patient samples were analyzed using the immuno-infrared sensor. Out of this collective, 100 patients were randomly selected for the present study. Infrared amide I data of Aβ extracted from CSF and blood plasma were adopted from (Nabers et al., Anal. Chem. Doi: 10.1021/acs.analchem.5b04286 (2016)).

### Solutions and reagents:

Phosphate buffered saline (PBS-buffer): 137 mM sodium chloride (NaCI), 2.7 mM potassium chloride (KCI), 12 mM total-phosphate (in the form of Na₂HPO₄ and NaH₂PO₄), pH 7.4.

Casein blocking-solution: 200 mM sodium hydroxide (NaOH), 1 % (w/v) casein from bovine milk (powder), pH adjusted with H₃PO₄ to 7.4.

Silanization-solution: The used NHS-silane (N-(4,4,4-triethoxysilanebutyl)succinamic acid 2,5-dioxopyrrolidin-1-yl ester) was synthesized and characterized as described (Schartner et al., JACS 135(10):4079-4087 (2013)).

Antibody: For the extraction of Aβ from the respective body fluid the antibody A8978 (lot no: 061M4773, Sigma Aldrich) was employed. In case of the Tau protein detection the antibody Tau-5 (AHB0042, Thermo Fisher Scientific) was used.

Performing the measurement: The general procedure is identical to the one described in WO 2015/121339. IR-measurements were performed on a Vertex 70V spectrometer (Bruker Optics GmbH, Ettlingen, Germany) with liquid nitrogen cooled mercury-cadmium-telluride (MCT) detector. Double-sided interferograms were recorded in forward-backward interferometer movement at a 80 kHz data rate with a spectral resolution of 2 cm⁻¹, Blackman-Harris-3-Term-apodisation, Mertz-phase correction and 4 times zero filling. Reference spectra were recorded as an average of 1000, sample spectra of 200 interferograms. Recording reference single channel spectra of the blank sensor, sensor with 2-propanol, the silanized surface, the buffers, antibody or casein coated surface in equilibrium states enabled high sensitivity difference spectroscopy based on Lambert-Beer law (E=-log(I/I₀). The absorbance of the state change is the negative decadic logarithm of the intensity relation before and after the change. Workflow: Details of the sensor preparation for the FTIR-spectroscopic analysis of the Tau protein in CSF were published previously (WO 2015/121339; Nabers et al., Anal. Chem. Doi:10.1021/acs.analchem.5b04286 (2016); Nabers et al., Journal of Biophotonics 9(3):224-34 doi:10.1002/jbio.201400145 (2016)). Briefly, the total volume of the flow-cell including all connection tubes amounted to 400 µl. For each analysis, one sensor element per sample was freshly functionalized with silane (Schartner et al., JACS 135(10):4079-87 doi:10.1021/ja400253p (2013)) and antibody. Before analysis the surface was saturated with a casein blocking solution. For Aβ detection in CSF and blood plasma, the monoclonal antibody A8978 (Sigma Aldrich, aa 13-28) was used. Tau capturing was provided by monoclonal Tau-5 antibody (Life Technology, aa 210-230). For the analysis 50 µl CSF or 150 µl of EDTA-plasma were added to the circulating buffer with a flow-rate of 1 ml/min, respectively. Pretreatment of the spectra: By scaled subtraction of a reference spectrum water vapor was removed. Spectra were baseline corrected.

Example 1: The Aβ and Tau protein secondary structure distribution for accurate DC and DAT differentiation.

The performed study included 300 samples from 61 DC and 39 DAT patients. Details about the patients differential diagnosis were described previously (Nabers et al., Anal. Chem. Doi: 10.1021/acs.analchem.5b04286 (2016). In general, the patient collective was separated into DCs and DAT subjects. The DAT group was further sub-classified into early, moderate, and severe states of Alzheimer's disease. For a small number of DC patients a complete differential diagnosis was available including patients suffer from dementia not due to Alzheimer's disease origin such as Parkinson disease or vascular dementia. For the analysis of the secondary structure distribution of Aβ and Tau in CSF and/or plasma, both biomarker were extracted from the respective fluid by an immuno-infrared sensor as described by Nabers et al. (Nabers et al., Anal. Chem. Doi: 10.1021/acs.analchem.5b04286 (2016)). Therefore, Aβ and Tau were separately captured out of the CSF or plasma by the surface immobilized monoclonal antibody A8978 (aa13-28 of Aβ) and Tau-5 (aa210-230), respectively. The secondary structure distribution was indicated by the recorded amide I maximum frequency of Aβ and Tau. In the previous study, a simple threshold classifier was established with a discriminative marker frequency of 1643 cm⁻¹ for DC and DAT differentiation. The same marker band was used within the current study. At first, the amide I maximum of Aβ from CSF was determined for each patient sample. Thereby, a maximum position below 1643 cm⁻¹ was indicative for DAT. Next, the amide I maximum of Aβ from blood EDTA-plasma was identified. Finally, we detected the maximum of the extracted Tau protein fraction in CSF. The discriminative marker band was identically defined for both biomarkers and both bodily fluids at <1643 cm⁻¹ indicative for DAT. The amide I maximum distribution of the DC and DAT group showed highly significant differences for Aβ from CSF (Kruskal-Wallis ANOVA; p=2.5^{∗}10⁻¹¹; confidence level β=0.05) and blood plasma (p=3.4^{∗}10⁻⁹) and significantly differed for Tau (p=1.6^{∗}10⁻³) from CSF (Fig. 2). Thus, a smaller shift of the Tau amide I maximum was observed for DAT subjects as compared to Aβ. The mean amide I maximum of Tau was 1644 cm⁻¹ for the DC and 1642 cm⁻¹ for the DAT group as compared to Aβ from CSF with 1645 cm⁻¹ for DC and 1641 cm⁻¹ for DAT subjects. Aβ from blood plasma revealed a mean maximum of 1648 cm⁻¹ for the DC and 1641 cm⁻¹ for the DAT group. Based on these distributions, also shown in a 3D-scatter plot in Fig. 3 with a transparent black box indicating DAT, the diagnostic performance of each biomarker by itself was calculated by giving the accuracy, sensitivity, and specificity. Additionally, Receiver Operating Characteristic- (ROC-) curve analyses were performed by scanning the threshold between 1630.5 cm⁻¹ to 1660.5 cm⁻¹ and determining the sensitivity and specificity at each wavenumber. Similar to the results of Nabers et al., the diagnostic accuracy of Aβ based analysis was highest with 90 % for CSF (Fig. 4A,D; specificity 89 %, sensitivity 92 %, AUC 0.90) as compared to the analysis of the Aβ secondary structure distribution in blood plasma with 85 % (Fig. 4B,D; specificity 90 %, sensitivity 77 %, 0.85). In contrast, DC and DAT differentiation based on the Tau protein secondary structure distribution in CSF only revealed a diagnostic accuracy of 68 % (Fig. 4C,D; specificity 67 %, sensitivity 69 %, AUC 0.67), indicating that Tau alone is not an appropriate biomarker for DAT detection. But by adding all three biomarker values to a majority vote classifier, thus making a diagnostic decision based the presented biomarker panel (two maxima below the threshold = diseased, two maxima above the threshold = non DAT), the diagnostic performance could be increased to a specificity of 95 %, sensitivity of 90 %, and thus to an overall accuracy of 93 % as compared to the clinical diagnosis. Therewith, only 3 false positives out of 61 DCs and 4 false negatives out of 31 DATs were identified.

Example 2: A combined assay for DC and DAT differential diagnostics.

The combined data analysis provided also the potential to sub classify both diagnostics groups. This is schematically shown in Fig. 5. For instance, Aβ from CSF and plasma demonstrates an amide I maximum above or equal to 1643 cm⁻¹, but the Tau amide I maximum is below 1643 cm⁻¹, in this case another type of dementia might be potentially indicated by the combined immuno-infrared assay (Fig. 5). In contrast, when the amide I maxima of Aβ from CSF and plasma are below the marker band but the Tau maximum is above, an early state of DAT will be displayed. This procedure was applied to both diagnostics groups within our study. The amide I maximum of Aβ from CSF demonstrated in 69 % of all DC cases a higher maximum value than Tau from CSF. This effect may be explained by higher disordered properties of the Tau protein as compared to Aβ. On the other hand, in 25 % of all DC cases the maximum was lower for Aβ and only in 6 % of all cases the maxima were identically. This relation completely changed for the DAT group. Therein only 31 % of all DAT patients yielded an amide I maximum for Aβ greater than for Tau, 62 % showed a lower Aβ maximum (Fig. 6). This supports the hypothesis that Aβ accumulation is in early and initiating event in Alzheimer's disease progression and accompanied by Tau protein aggregation.

## Claims

1. A method for the differential diagnosis and sub-classification of Alzheimer's disease into different disease stages by direct analysis of the secondary structure distribution of a soluble Amyloid-beta (Aβ) peptide fraction and a soluble Tau protein fraction in bodily fluids, comprising the steps
(a) conducting, in a first IR cell comprising a first infrared sensor element having an internal reflection element with a core of an infrared transparent material and at least one receptor for the Aβ peptide directly grafted to at least one surface of said core, said at least one receptor for the Aβ peptide being antibodies capable of specific and conformationally independent binding to the Aβ peptide, at least one flux of a body fluid with soluble Aβ peptide, submitting an IR beam through said first IR cell, and obtaining an infrared spectrum therefrom;
(b) conducting, in a second IR cell comprising a second infrared sensor element having an internal reflection element with a core of an infrared transparent material and at least one receptor for the Tau protein directly grafted to at least one surface of said core, said at least one receptor for the Tau protein being antibodies capable of specific and conformationally independent binding to the Tau protein, at least one flux of a body fluid with soluble Tau protein, submitting an IR beam through said second IR cell, and obtaining an infrared spectrum therefrom; and
(c) analyzing the obtained infrared spectra to determine the secondary structure distribution of the soluble Aβ peptide and of the soluble Tau protein in the bodily fluids for the differential diagnosis, wherein a downshift of the amide I band of the Aβ peptide and of the Tau protein is indicative for the disease stage.

2. The method of claim 1, wherein
(i) the infrared transparent material of the first and second IR cell is independently selected from silicon, germanium, zinc selenide, gallium selenide and diamond, and preferably is germanium; and/or
(ii) the blocking substance not cross-reacting with the Aβ peptide or the Tau protein is selected from casein, ethanolamine, L-lysine, polyethylene glycols, albumins and derivatives thereof.

3. The method of claim 1, wherein said first and second infrared sensor elements comprise a germanium internal reflection element being of trapezoid or parallelogram shape.

4. The method of claim 3, wherein the internal reflection element
(i) is a germanium monocrystal, preferably is a trapezoid cut germanium monocrystal; and/or
(ii) provides for more than one passages of the infrared light through the reflection element; and/or
(iii) is further suitable for the alternative or parallel analysis by another optical method including detection of fluorescence at different wavelengths.

5. The method of any one of claims 1 to 4, wherein
(i) the receptor binding to the Aβ peptide is an antibody specifically binding to the central epitope of the Aβ peptide, including antibody A8978; or
(ii) the receptor binding to the Tau protein is an antibody specifically binding to the middle epitope of Tau and to an epitope present in all Tau variants, including antibody Tau-5.

6. The method of any one of claims 1 to 5, wherein the method provides the
differentiation of Alzheimer's disease into early/prodromal, moderate, and severe disease stages, and wherein
(i) amide I maxima of the mentioned biomarkers, Aβ from CSF, Aβ from blood plasma, and Tau from CSF, are all below the discriminative threshold (1643 cm⁻¹ ± 5 cm⁻¹) which are indicative for a severe disease stage,
(ii) amide I maxima of two biomarkers, one is Aβ from CSF or blood plasma and the other one is Tau from CSF, below the discriminative threshold (1643 cm⁻¹ ± 5 cm⁻¹) are indicative for a moderate disease stage,
(iii) amide I maxima of one or two biomarkers (Aβ from CSF and/or blood plasma), but not Tau from CSF, below the discriminative threshold (1643 cm⁻¹ 5 cm⁻¹) are indicative for an early disease stage.

7. The method of any one of claims 1 to 5, wherein the differential diagnosis
provides for an assured clinical profile of the dementia type, preferably the method comprises the detection of the secondary structure distribution of Aβ from CSF (A), Aβ from blood plasma (B), and Tau from CSF (C), most preferably the method enables the differential diagnosis of Dementia Alzheimer type (DAT) and a Disease Control (DC),
wherein DAT patients being sub-classified into early, moderate, and severe DAT, and DC patients being separated into healthy controls, other diseases, and dementia due to another origin than Alzheimer's disease.

8. The method of claim 7, wherein
(i) for both biomarkers, (A) and (B) for Aβ and (C) for Tau, a discriminative threshold (1643 cm⁻¹ ± 5 cm⁻¹) separates Alzheimer's disease and DC patients; and/or
(ii) the combination of (A), (B), and (C) provides a biomarker panel applicable for an assured DAT diagnosis.

9. The method of any one of claims 1 to 8, which provides information about the
patient disease state based on the analysis of bodily fluids, wherein a shift of the amide I band maximum of the biomarker protein is a classifier indicative for the progression of the disease.

10. The method of claim 9, wherein a threshold classifier with a value of 1638-1648 cm⁻¹ is a classifier indicative for the progression of the disease.

11. A kit for the differential diagnosis and sub classification of Alzheimer's disease into different disease stages comprising a first and second infrared sensor element as defined in any one of claims 1 to 5.

12. A device for the differential diagnosis and sub classification of Alzheimer's disease into different disease stages, said device comprising a first and second infrared sensor element as defined in any one of claims 1 to 5.

13. Use of the first and second infrared sensor element of any one of claims 1 to 5, the kit of claim 11 or the device of claim 12 for direct analysis of the secondary structure distribution of a soluble Amyloid-beta (Aβ) peptide fraction and a soluble Tau protein fraction in bodily fluids.

## Patentansprüche

1. Verfahren zur Differentialdiagnose und Subklassifizierung der Alzheimer-Krankheit in verschiedene Krankheitsstadien durch direkte Analyse der Sekundärstrukturverteilung einer löslichen Amyloid-beta (Aß)-Peptidfraktion und einer löslichen Tau-Proteinfraktion in Körperflüssigkeiten, umfassend die Schritte
(a) Durchleiten mindestens einer Strömung einer Körperflüssigkeit mit löslichem Aβ -Peptid in eine erste IR-Zelle, die ein erstes Infrarot-Sensorelement mit einem internen Reflektionselement mit einem Kern aus einem Infrarottransparenten Material und mindestens einem Rezeptor für das Aß-Peptid umfasst, das direkt auf mindestens eine Oberfläche des Kerns gegrafted ist, wobei der mindestens eine Rezeptor für das Aß-Peptid Antikörper sind, die zur spezifischen und konformationsunabhängigen Bindung an das Aß-Peptid fähig sind,
Senden eines IR-Strahls durch die erste IR-Zelle und Erhalten eines Infrarotspektrums davon;
(b) Durchleiten mindestens einer Strömung einer Körperflüssigkeit mit löslichem Tau-Protein in einer zweiten IR-Zelle, die ein zweites Infrarot-Sensorelement mit einem internen Reflektionselement mit einem Kern aus einem Infrarottransparenten Material und mindestens einem Rezeptor für das Tau-Protein, der direkt auf mindestens eine Oberfläche des Kerns gegrafted ist, umfasst, wobei der mindestens eine Rezeptor für das Tau-Protein Antikörper sind, die zu einer spezifischen und konformationsunabhängigen Bindung an das Tau-Protein fähig sind,
Leiten eines IR-Strahls durch die zweite IR-Zelle und Erhalten eines Infrarotspektrums davon; und
(c) Analysieren der erhaltenen Infrarotspektren, um die Sekundärstrukturverteilung des löslichen Aß-Peptids und des löslichen Tau-Proteins in den Körperflüssigkeiten für die Differentialdiagnose zu bestimmen, wobei eine Abwärtsverschiebung der Amid-I-Bande des Aß-Peptids und des Tau-Proteins indikativ für das Krankheitsstadium ist.

2. Das Verfahren nach Anspruch 1, wobei
(i) das Infrarot-transparente Material der ersten und zweiten IR-Zelle unabhängig voneinander aus Silizium, Germanium, Zinkselenid, Galliumselenid und Diamant ausgewählt wird und vorzugsweise Germanium ist; und/oder
(ii) die blockierende Substanz, die nicht mit dem Aß-Peptid oder dem Tau-Protein kreuzreagiert, ausgewählt ist aus Casein, Ethanolamin, L-Lysin, Polyethylenglykolen, Albuminen und Derivaten davon.

3. Das Verfahren nach Anspruch 1, wobei die ersten und zweiten Infrarot-Sensorelemente ein Germanium-Innenreflektionselement in Form eines Trapezes oder Parallelogramms umfassen.

4. Das Verfahren nach Anspruch 3, wobei das interne Reflektionselement
(i) ein Germanium-Monokristall, vorzugsweise ein trapezförmig geschnittener Germanium-Monokristall ist; und/oder
(ii) für mehr als einen Durchgang des Infrarotlichts durch das Reflektionselement sorgt; und/oder
(iii) ferner für die alternative oder parallele Analyse durch ein anderes optisches Verfahren einschließlich der Erfassung von Fluoreszenz bei verschiedenen Wellenlängen geeignet ist.

5. Das Verfahren nach einem der Ansprüche 1 bis 4, wobei
(i) der an das Aß-Peptid bindende Rezeptor ein Antikörper ist, der spezifisch an das zentrale Epitop des Aß-Peptids bindet, einschließlich des Antikörpers A8978; oder
(ii) der an das Tau-Protein bindende Rezeptor ein Antikörper ist, der spezifisch an das mittlere Epitop von Tau und an ein Epitop bindet, das in allen Tau-Varianten vorhanden ist, einschließlich des Antikörpers Tau-5.

6. Das Verfahren nach einem der Ansprüche 1 bis 5, wobei das Verfahren die Differenzierung der Alzheimer-Krankheit in frühe/prodromale, moderate und schwere Krankheitsstadien ermöglicht, und wobei
(i) Amid-I-Maxima der erwähnten Biomarker, Aβ aus Liquor, Aβ aus Blutplasma und Tau aus Liquor, alle unterhalb der Unterscheidungsgrenze (1643 cm⁻¹ ± 5 cm⁻¹) liegen, was auf ein schweres Krankheitsstadium hinweisen,
(ii) Amid-I-Maxima von zwei Biomarkern, von denen einer Aβ aus Liquor oder Blutplasma und der andere Tau aus Liquor ist, unterhalb der Unterscheidungsgrenze (1643 cm⁻¹ ± 5 cm⁻¹) liegen, was auf ein mittleres Krankheitsstadium hindeutet,
(iii) Amid-I-Maxima von einem oder zwei Biomarkern (Aβ aus Liquor und/oder Blutplasma), aber nicht Tau aus Liquor, unterhalb der Unterscheidungsgrenze (1643 cm⁻¹ ± 5 cm⁻¹) auf ein frühes Krankheitsstadium hinweisen.

7. Das Verfahren nach einem der Ansprüche 1 bis 5, wobei die Differentialdiagnose ein gesichertes klinisches Profil des Demenztyps liefert, vorzugsweise umfasst das Verfahren den Nachweis der Sekundärstrukturverteilung von Aβ aus Liquor (A), Aβ aus Blutplasma (B) und Tau aus Liquor (C), am meisten bevorzugt ermöglicht das Verfahren die Differentialdiagnose von Demenz vom Alzheimer-Typ (DAT) und einer Kontrollerkrankung (DC),
wobei DAT-Patienten in frühe, mittelschwere und schwere DAT unterteilt werden und DC-Patienten in gesunde Kontrollen, andere Krankheiten und Demenz aufgrund einer anderen Ursache als der Alzheimer-Krankheit unterteilt werden.

8. Das Verfahren nach Anspruch 7, wobei
(i) für beide Biomarker, (A) und (B) für Aβ und (C) für Tau, eine Unterscheidungsgrenze (1643 cm⁻¹ ± 5 cm⁻¹) Alzheimer-Krankheit und DC-Patienten trennt; und/oder
(ii) die Kombination von (A), (B) und (C) ein Biomarker-Panel liefert, das für eine gesicherte DAT-Diagnose geeignet ist.

9. Das Verfahren nach einem der Ansprüche 1 bis 8, das auf der Grundlage der Analyse von Körperflüssigkeiten Informationen über den Krankheitszustand des Patienten liefert, wobei eine Verschiebung des Amid-I-Bandenmaximums des Biomarkerproteins ein Klassifikator ist, der auf das Fortschreiten der Krankheit hinweist.

10. Das Verfahren nach Anspruch 9, wobei ein Schwellenwert-Klassifikator mit einem Wert von 1638-1648 cm⁻¹ ein Klassifikator ist, der auf das Fortschreiten der Krankheit hindeutet.

11. Kit für die Differentialdiagnose und Subklassifizierung der Alzheimer-Krankheit in verschiedene Krankheitsstadien, umfassend ein erstes und ein zweites Infrarot-Sensorelement gemäß einem der Ansprüche 1 bis 5.

12. Vorrichtung zur Differentialdiagnose und Subklassifizierung der Alzheimer-Krankheit in verschiedene Krankheitsstadien, wobei die Vorrichtung ein erstes und ein zweites Infrarot-Sensorelement gemäß einem der Ansprüche 1 bis 5 umfasst.

13. Verwendung des ersten und zweiten Infrarot-Sensorelements nach einem der Ansprüche 1 bis 5, des Kits nach Anspruch 11 oder der Vorrichtung nach Anspruch 12 zur direkten Analyse der Sekundärstrukturverteilung einer löslichen Amyloid-beta (Aß)-Peptidfraktion und einer löslichen Tau-Proteinfraktion in Körperflüssigkeiten.

## Revendications

1. Procédé pour le diagnostic différentiel et la sous-classification de la maladie d'Alzheimer en différents stades de maladie par analyse directe de la distribution de structure secondaire d'une fraction de peptide amyloïde bêta (Aβ) soluble et d'une fraction de protéine Tau soluble dans des fluides corporels, comprenant les étapes de
(a) conduction, dans une première cellule IR comprenant un premier élément de capteur infrarouge comportant un élément de réflexion interne avec un noyau composé d'un matériau transparent à l'infrarouge et au moins un récepteur pour le peptide Aβ directement greffé à au moins une surface dudit noyau, ledit au moins un récepteur pour le peptide Aβ étant des anticorps capables de se lier spécifiquement et de manière conformationnellement indépendante au peptide Aβ, d'au moins un flux d'un fluide corporel avec du peptide Aβ soluble, soumission d'un faisceau IR à travers ladite première cellule IR, et obtention d'un spectre infrarouge à partir de celui-ci ;
(b) conduction, dans une deuxième cellule IR comprenant un deuxième élément de capteur infrarouge comportant un élément de réflexion interne avec un noyau composé d'un matériau transparent à l'infrarouge et au moins un récepteur pour la protéine Tau directement greffé à au moins une surface dudit noyau, ledit au moins un récepteur pour la protéine Tau étant des anticorps capables de se lier spécifiquement et de manière conformationnellement indépendante à la protéine Tau, d'au moins un flux d'un fluide corporel avec de la protéine Tau soluble, soumission d'un faisceau IR à travers ladite deuxième cellule IR, et obtention d'un spectre infrarouge à partir de celui-ci ; et
(c) analyse des spectres infrarouges obtenus pour déterminer la distribution de structure secondaire du peptide Aβ soluble et de la protéine Tau soluble dans les fluides corporels pour le diagnostic différentiel, sachant qu'un décalage vers le bas de la bande d'amide I du peptide Aβ et de la protéine Tau est indicateur du stade de maladie.

2. Le procédé de la revendication 1, sachant que
(i) le matériau transparent à l'infrarouge de la première et de la deuxième cellule IR est sélectionné indépendamment parmi le silicium, le germanium, le séléniure de zinc, le séléniure de gallium et le diamant, et est de préférence le germanium ; et/ou
(ii) la substance de blocage n'effectuant pas de réaction croisée avec le peptide Aβ ou la protéine Tau est sélectionnée parmi la caséine, l'éthanolamine, la L-lysine, les glycols de polyéthylène, les albumines et les dérivés de ceux-ci.

3. Le procédé de la revendication 1, sachant que ledit premier et ledit deuxième élément de capteur infrarouge comprennent un élément de réflexion interne en germanium qui est de forme trapézoïdale ou en forme de parallélogramme.

4. Le procédé de la revendication 3, sachant que l'élément de réflexion interne
(i) est un monocristal de germanium, de préférence est un monocristal de germanium à découpe trapézoïdale ; et/ou
(ii) assure plus d'un passage de la lumière infrarouge à travers l'élément de réflexion ; et/ou
(iii) convient en outre pour l'analyse alternative ou parallèle par un autre procédé optique incluant la détection de fluorescence à différentes longueurs d'onde.

5. Le procédé de l'une quelconque des revendications 1 à 4, sachant que
(i) le récepteur qui se lie au peptide Aβ est un anticorps qui se lie spécifiquement à l'épitope central du peptide Aβ, incluant l'anticorps A8978 ; ou
(ii) le récepteur qui se lie à la protéine Tau est un anticorps qui se lie spécifiquement à l'épitope médian de Tau et à un épitope présent dans tous les variants de Tau, incluant l'anticorps Tau-5.

6. Le procédé de l'une quelconque des revendications 1 à 5,
sachant que le procédé fournit la différenciation de la maladie d'Alzheimer en stades précoce/prodromal, modéré, et grave, et sachant que
(i) des maxima d'amide I des biomarqueurs mentionnés, Aβ provenant de CSF, Aβ provenant de plasma sanguin, et Tau provenant de CSF, qui sont tous sous le seuil discriminatoire (1643 cm⁻¹ ± 5 cm⁻¹) sont indicateurs d'un stade de maladie grave,
(ii) des maxima d'amide I de deux biomarqueurs, l'un étant Aβ provenant de CSF ou de plasma sanguin et l'autre étant Tau provenant de CSF, sous le seuil discriminatoire (1643 cm⁻¹ ± 5 cm⁻¹) sont indicateurs d'un stade de maladie modéré,
(iii) des maxima d'amide I d'un ou de deux biomarqueurs (Aβ provenant de CSF et/ou de plasma sanguin), mais pas Tau provenant de CSF, sous le seuil discriminatoire (1643 cm⁻¹ ± 5 cm⁻¹) sont indicateurs d'un stade de maladie précoce.

7. Le procédé de l'une quelconque des revendications 1 à 5,
sachant que le diagnostic différentiel fournit un profil clinique sûr du type de démence, de préférence le procédé comprend la détection de la distribution de structure secondaire d'Aβ provenant de CSF (A), d'Aβ provenant de plasma sanguin (B), et de Tau provenant de CSF (C), de manière la plus préférentielle le procédé permet le diagnostic différentiel du type de démence Alzheimer (DAT) et d'un témoin de maladie (DC),
sachant que les patients DAT sont sous-classifiés en DAT précoce, modéré, et grave, et les patients DC sont distingués en témoins sains, autres maladies, et démence due à une autre origine que la maladie d'Alzheimer.

8. Le procédé de la revendication 7, sachant que
(i) pour les deux biomarqueurs, (A) et (B) pour Aβ et (C) pour Tau, un seuil discriminatoire (1643 cm⁻¹ ± 5 cm⁻¹) distingue les patients ayant la maladie d'Alzheimer et les patients DC ; et/ou
(ii) la combinaison de (A), (B), et (C) fournit un panel de biomarqueurs applicable pour un diagnostic DAT sûr.

9. Le procédé de l'une quelconque des revendications 1 à 8,
lequel fournit des informations sur l'état de maladie d'un patient sur la base de l'analyse de fluides corporels, sachant qu'un décalage du maximum de bande d'amide I de la protéine biomarqueur est un classificateur indicateur de la progression de la maladie.

10. Le procédé de la revendication 9,
sachant qu'un classificateur seuil d'une valeur de 1638-1648 cm⁻¹ est un classificateur indicateur de la progression de la maladie.

11. Kit pour le diagnostic différentiel et la sous-classification de la maladie d'Alzheimer en différents stades de maladie comprenant un premier et un deuxième élément de capteur infrarouge tels que définis dans l'une quelconque des revendications 1 à 5.

12. Dispositif pour le diagnostic différentiel et la sous-classification de la maladie d'Alzheimer en différents stades de maladie, ledit dispositif comprenant un premier et un deuxième élément de capteur infrarouge tels que définis dans l'une quelconque des revendications 1 à 5.

13. Utilisation du premier et du deuxième élément de capteur infrarouge de l'une quelconque des revendications 1 à 5, du kit de la revendication 11 ou du dispositif de la revendication 12 pour l'analyse directe de la distribution de structure secondaire d'une fraction de peptide amyloïde bêta (Aβ) soluble et d'une fraction de protéine Tau soluble dans des fluides corporels.
